# EUROPEAN PATENT SPECIFICATION

(11) **EP 2 509 640 B1**
(45) Date of publication and mention of the grant of the patent: **03.05.2017**
(21) Application number: 09852002.6
(22) Date of filing: 07.12.2009
(51) Int. Cl.: A61L 2/18, A01N 31/02, A61L 101/36

(54) **TREATMENT OF MEAT PROCESSING CUTTING TOOLS WITH BIOCIDE**
BEHANDLUNG VON SCHNEIDWERKZEUGEN ZUR FLEISCHVERARBEITUNG MIT EINEM BIOZID
TRAITEMENT D'OUTILS DE DÉCOUPE POUR TRANSFORMATION DE VIANDE AVEC UN BIOCIDE

(43) Date of publication of application: 17.10.2012
(73) Proprietor: Ecolab Inc., St. Paul, MN 55102 (US)
(72) Inventor: VERKAAR, Edward L.C., NL-5991 LW Baarlo (NL); KLOOTE, Paul, NL-5241 KP Rosmalen (NL); NATEN, Stefan, B-1852 Beigem (BE)
(74) Representative: Godemeyer Blum Lenze Patentanwälte Partnerschaft mbB - werkpatent
(86) International application number: PCT/IB2009/055551
(87) International publication number: WO 2011/070392

(56) References cited:
- EP-A1- 1 495 770
- US-A- 5 324 477
- US-A1- 2002 119 743
- US-A1- 2002 192 340
- US-A1- 2005 152 991
- US-A1- 2005 159 324
- US-A1- 2007 010 420
- AZANZA M.P.V.: 'Hydrogen peroxide, ..........' FAO 2004, [Online] 2004, XP003028109 Retrieved from the Internet: <URL:ftp://ftp.fao.org/es/esn/jecfa/cta/CTA _63_Antimicrobials.pdf>

## Description

### Field of the Invention

The invention relates to compositions including peroxyacetic acid and peroxyoctanoic acid and methods for reducing microbial contamination on cutting tools and instruments when processing carcasses and meats. The methods include the step of applying a mixed peroxycarboxylic acid composition to the cutting instruments and tools.

### Background of the Invention

All carcasses entering the processing environment are contaminated with bacteria, some with pathogenic bacteria such as Salmonella or spoilage-causing bacteria such as Pseudomonas. Contaminated meat, fecal matter and dirt are the main sources of this contamination. As a result of such contamination, carcasses are typically washed at any of several steps during the process of converting a live animal to an edible food product. Such washing aims to remove dirt, offal, blood, viscera, other debris, and microbes from the animal. Removing or reducing microbes aids the safe storage and consumption of poultry and meat, yet many existing washing procedures fail to significantly reduce the microbe burden on poultry and/or meats. In particular, the potential for poultry skin to become cross-contaminated is worsened by the ability of all types of bacteria (Gram-positive, Gram-negative, flagellated, non-flagellated, rods or cocci) to adhere within only 15 seconds of contact. Once in the processing environment, a significant number of carcasses become cross-contaminated with pathogens during handling, scalding, mechanical processing, and chilling. Current methods for many of these procedures also fail to significantly reduce the microbe burden on poultry.

The cutting instruments and knives used to process poultry or other carcasses are particularly suited for contamination since they contact the carcass that is often contaminated. The cutting tools provide an unwanted opportunity to cross-contaminate otherwise "clean" carcasses. These microbes can potentially grow on and contaminate other carcasses and processing equipment resulting in e.g. E. coli, Salmonella, enterobacteria, and/or Campylobacter contamination or cross contamination during meat processing. As one skilled in the art will recognize, Salmonella and other microorganisms are generally undesirable to the food product and more importantly to humans. Some pathogenic and spoilage causing bacteria (such as Listeria monocytogenes or E.coli) can cause buildup of slime or biofilm on all contact surfaces, which requires frequent cleaning to remove.

Many countries require hot water disinfection of cutting tools. For the European Union, this is mandated in EU regulation 853/2004 (Hygiene Regulation). In order to have full disinfection of cutting tools with hot water of 82°C, the respective tools need to be in contact with hot water long enough to heat up the metallic surface. Depending on the size of the cutting tool, this level is achieved after at least 10 seconds. For slaughter robots this means that the cutting tools need to be rinsed with large volumes of hot water or immersed in the hot water with higher dwell times. For knives this means longer dip times in the sterilizers.

Microbial contamination or cross contamination of poultry or meat via water continues to be a major concern for poultry and meat processors and end users. Ideally, an antimicrobial agent or compound used in such a system will have several important properties in addition to its antimicrobial efficacy. The compound or agent should have no technical effect on the final food product. Residual activity implies the presence of a film of antimicrobial material which will continue to have antimicrobial effect which may require further rinsing of the food product. The antimicrobial agent preferably should also be odor free to prevent transfer of undesirable odors onto food stuffs. The biocidal agent or biocide should also be composed of approved food additive materials which will not affect food wholesomeness, nor affect humans should incidental ingestion result. In addition, the biocidal agent should ideally be composed of naturally occurring or innocuous ingredients, which are chemically compatible with the environment and cause no concerns for toxic residues within the water.

For decades the use of substances other than potable water for cleaning carcasses, that is antimicrobial substances, has been resisted because it was feared such antimicrobial substances would mask unhygienic slaughter or improper processing practices and would certainly not be an incentive for businesses to implement hygienic practices. If permitted, it was feared that their widespread use coupled with high bacterial counts due to unhygienic practices, would induce resistance of the micro flora present on the surface of the treated products. European standards require that meat-producing and processing establishments must disinfect cutting tools with hot water supplied at not less than 82°C. However, several European member states have voiced concern that disinfection at these temperatures may lead to protein coagulates and that such coagulates may result in an inefficient disinfection of knives. As a result of this objection, an opinion by the Scientific Committee on the Veterinary Measures relating to Public Health on the cleaning and disinfection of knives evaluated several options and concluded that alternatives to hot water disinfection should be possible. As a result, hygiene regulation 853/2004/EEC of the European Parliament and of the Council of 29 April 2004 specified hygiene rules for food of animal origin clearly stating that food business operators should have facilities for disinfecting tools with hot water of 82°C or an alternative system with equivalent effect. In practice, that requirement exposes the personnel involved to unfavourable working conditions due to release of steam and heat at the site of operation. Furthermore, coagulation of protein and fat as a result of hot water disinfection will not eliminate the build-up of biofilms but rather will enhance it.

In the past, poultry and meat have generally been treated with chlorinated compounds, organic acids, acidified sodium chlorite, trisodium phosphate, or ozone. Generally, these materials are effective in reducing microbial contamination on the meat's surfaces. However, the use rate of these antimicrobials is very high because they are not effective at low concentrations or they tend to be rapidly consumed by the high organic load included with the meat and poultry. Excessive chlorination of food processing water with hypochlorite has prompted concern over production of toxic or carcinogenic organochlorine compounds and other by-products.

The EPA approved a peroxyacetic acid-based composition in 1996 for controlling microbial growth and reducing biofilm formation in fruit and vegetable transport or process waters. From a historical perspective, peroxyacetic acid has been used for food contact surface sanitizing, aseptic packaging and medical device cold-sterilization. In addition to its biocidal properties, the environmentally-friendly decomposition byproducts and good stability in the presence of organic matter helped gain acceptance of this technology among fruit and vegetable packers, handlers, and processors.

US 2005/0152991 A1 (D1)
Antimicrobial compositions including medium chain peroxycarboxylic acids and their use for cleaning or sanitizing of equipment in the food service of food-processing industry.

US 2007/0010420 A1 (D2)
relates to compositions including surfactant peroxycarboxylic acid and their use in the cleaning or sanitizing of equipment in the food service of processing industries.

US 2005/0159324 A1 (D3)
relates to methods for reducing microbial contamination on poultry or on surfaces used in processing poultry using compositions including medium chain peroxycarboxylic acids.

US 5,324,477 A (D4)
relates to a process for disinfecting hard surfaces under water-hardness-stabilized and noncorrosive conditions with chlorine dioxide produced in solution by mixing of a solution of sodium chlorite and another acidic component in a generator.

Nevertheless, there remains a need for an improved method of sanitizing meat processing equipment to ensure the cutting instruments and tools used therein are free of microbes. In particular, there remains a need for an improved method of cleaning and sanitizing knives used in poultry and meat processing.

### Summary

A method of reducing microbes on cutting tools is disclosed. The method provides a biocide for applying to cutting tools that has a high degree of antimicrobial efficacy and that is safely ingestible by humans while imposing no unacceptable environmental incompatibility. This method includes applying to cutting tools a biocide comprised of an organic acid containing a fatty acid at concentration of at least 0.15 wt % according to claim 1. Use of the proposed method may lead to lower water consumption. In an embodiment, the organic acid containing a fatty acid includes peroxycarboxylic acids. In another embodiment the organic acid containing a fatty acid includes peroxyacetic acid and peroxyoctanoic acid. The method may further include adding an optional additional organic acid and a scale inhibitor. In one embodiment the method is practiced before and/or during poultry
meat or other carcass processing. In yet another embodiment the biocide used in the method of the invention is comprised of peroctanoic acid, peroxyacid, octanoic acid, acetic acid, hydrogen peroxide, and hydroxyethylidene diphosphonic acid.

A biocide useful in the method of the present invention includes acetic acid, octanoic acid, peroxyacetic acid, peroxyoctanoic acid, and hydrogen peroxide. In one embodiment, an antimicrobial concentrate composition of the present invention includes about 40 to about 70 weight-% acetic acid, about 2 to about 20 weight-% octanoic acid, and about 5 to about 15 weight-% hydrogen peroxide. In another embodiment, the antimicrobial concentrate composition of the present invention includes an equilibrium mixture resulting from a combination of about 40 to about 70 weight-% acetic acid, about 2 to about 20 weight-% octanoic acid, and about 5 to about 15 weight-% hydrogen peroxide. In a third embodiment, the antimicrobial concentrate composition of the present invention includes about 30 to about 60 weight-% acetic acid, about 1 to about 15 weight-% octanoic acid, about 2 to about 12 weight-% hydrogen peroxide, about 6 to about 16 weight-% peroxyacetic acid, and about 0.1 to about 5 weight-% peroxyoctanoic acid.

In one embodiment, a biocidal composition useful in the method of the invention includes about 5 to about 1000 ppm acetic acid, about 0.5 to about 100 ppm octanoic acid, about 1 to about 200 ppm hydrogen peroxide, about 2 to about 300 ppm peroxyacetic acid, and about 0.1 to about 20 ppm peroxyoctanoic acid.

The biocide can be applied by methods including submersing, rinsing, and spraying and may be applied in either liquid or gaseous form. Mixed peroxycarboxylic acid antimicrobial compositions for use in the methods of the invention include mixtures of peroxyacetic acid and peroxyoctanoic acid. These methods include applying to the cutting tools and instruments before or during poultry, meat or carcass processing a mixed peroxycarboxylic acid biocidal composition in an amount and time sufficient to reduce the microbial population. Carcasses suitable for processing using the methods of the present invention include but are not limited to poultry, pig, lamb, goat, and cattle.

### Brief Description of the Drawings

Figure 1 is a graph showing comparative microbiological results for mesophilic bacteria using two different parameters (one run twice) on various locations of various cutting instruments.
Figure 2 is a graph showing comparative microbiological results for mesophilic bacteria using two different parameters (one run twice) on various locations of a carcass splitter.
Figure 3 is a graphical representation of the count for mesophilic bacteria on a bung dropper using two different parameters (one run twice) in the morning, at lunch break, and at the end of the day.
Figure 4 is a graphical representation of the count for mesophilic bacteria on a carcass splitter using two different parameters (one run twice) in the morning, at lunch break, and at the end of the day.
Figure 5 is a graphical representation of enterobacteriaceae taken using two different parameters (one run twice) various locations of various cutting instruments.
Figure 6 is a graph showing comparative microbiological results for enterobacteriaceae count using two different parameters (one run twice) on various locations of a carcass splitter.
Figure 7 is a graph showing comparative microbiological results for enterobacteriaceae count on the bung dropper using two different parameters (one run twice) taken in the morning, at lunch break, and at the end of the day.
Figure 8 is a graph showing comparative microbiological results for enterobacteriaceae count on a carcass splitter using two different parameters (one run twice) taken in the morning, at lunch break, and at the end of the day.
Figure 9 contains three graphs. The graphs are representations of the average mesophilic, and *enterobacteriaceae* bacteria count on carcasses taken using two different parameters (one run twice). A third graph represents the incidence of *Salmonella* presence on carcasses taken when running the three different tests.

### Detailed Description of the Invention

The term, "peroxy acid" is used interchangeably with the terms "peroxyacid," and "peracid" all of which describe an acid in which an acidic -OH group has been replaced by an -OOH group.

As used herein, the term, "biocide" or "biocidal composition" refers to a composition capable of killing microorganisms. In the case of the invention a biocidal composition is useful in reducing the microbial load or microbial contamination of an object to which it is applied.

As used herein, the phrase "food product" includes any food substance that might require treatment with a biocide composition and that is edible with or without further preparation. Food products include meat, poultry, fruits and vegetables. The term "produce" refers to food products such as fruits and vegetables and plants or plant-derived materials that are typically sold uncooked and, often, unpackaged, and that can sometimes be eaten raw. As used herein, the term "about" modifying the quantity of an ingredient in the compositions of the invention or employed in the methods of the invention refers to variation in the numerical quantity that can occur, for example, through typical measuring and liquid handling procedures used for making concentrates or use solutions in the real world; through inadvertent error in these procedures; through differences in the manufacture, source, or purity of the ingredients employed to make the compositions or carry out the methods; and the like. Whether or not modified by the term "about", the claims include equivalents to the quantities.

Differentiation of antimicrobial or bio- "-cidal" or "-static" activity, the definitions that describe the degree of efficacy, and the official laboratory protocols for measuring this efficacy are considerations for understanding the relevance of antimicrobial agents and compositions. Antimicrobial compositions can affect two kinds of microbial cell damage. The first is a lethal, irreversible action resulting in complete microbial cell destruction or incapacitation. The second type of cell damage is reversible, such that if the organism is rendered free of the agent, it can again multiply. The former is termed bactericidal (or biocidal) and the latter, bacteriostatic. A sanitizer and a disinfectant are, by definition, agents that provide antibacterial or bactericidal or biocidal activity. In contrast, a preservative is generally described as an inhibitor or bacteriostatic composition.

A biocide is a "processing aid" meaning that it is a substance that is not consumed as a food ingredient by itself but is intentionally used in the processing of raw materials, foods or their ingredients, to fulfill a certain technological purpose during treatment or processing and which may result in the unintentional but technically unavoidable presence of residues of the substance or its derivatives in the final product, provided that these residues do not present any health risk and do not have any technological effect on the finished product. During manufacture of food products, certain technically unavoidable residues may be left in the respective food product. It is important that these residues should not alter organoleptic properties of the final product nor should these residues become a health issue.

In an embodiment the biocide used in the method of reducing microbes on cutting tools includes less than 220 parts per million (ppm) peroxyacids such as peroxyacetic acid less than 110 ppm hydrogen peroxide, and less than 13 ppm 1-hydroxyethylidene-1,1-diphosphonic acid (HEDP) when applied to poultry and meat carcasses, poultry and meat parts, and organs. This limit is in accordance with current industry standards of good manufacturing practiced in the United States.

### Composition of Carboxylic Acids and Peroxycarboxylic Acids

Among other constituents, the method of the present invention employs a composition including a carboxylic acid. Generally, carboxylic acids have the formula R-COOH wherein the R can represent any number of different groups including aliphatic groups, alicyclic groups, aromatic groups, heterocyclic groups, all of which can be saturated or unsaturated as well as substituted or unsubstituted. Carboxylic acids also occur having one, two, three, or more carboxyl groups.

Peroxycarboxylic (or percarboxylic) acids generally have the formula R(CO₃H) ₙ, where R is an alkyl, arylalkyl, cycloalkyl, aromatic or heterocyclic group, and n is one, two, or three, and named by prefixing the parent acid with peroxy. While peroxycarboxylic acids are not very stable, their stability generally increases with increasing molecular weight. Thermal decomposition of these acids can generally proceed by free radical and nonradical paths, by photodecomposition or radical-induced decomposition, or by the action of metal ions or complexes. Percarboxylic acids can be made by the direct, acid catalyzed equilibrium action of 30-98 wt. % hydrogen peroxide with the carboxylic acid, by autoxidation of aldehydes, or from acid chlorides, and hydrides, or carboxylic anhydrides with hydrogen or sodium peroxide.

Typically the methods of the present invention include compositions including peroxyacetic acid. Peroxyacetic (or peracetic) acid is a peroxy carboxylic acid having the formula: CH₃COOOH. Generally, peroxyacetic acid is a liquid having an acrid odor at higher concentrations and is freely soluble in water, alcohol, ether, and sulfuric acid. Peroxyacetic acid can be prepared through any number of methods known to those of skill in the art including preparation from acetaldehyde and oxygen in the presence of cobalt acetate. A solution of peroxyacetic acid can be obtained by combining acetic acid with hydrogen peroxide. A 50% solution of peroxyacetic acid can be obtained by combining acetic anhydride, hydrogen peroxide and sulfuric acid. Other methods of formulation of peroxyacetic acid include those disclosed in U.S. Pat. No. 2,833,813, which is incorporated herein by reference in its entirety for all purposes.

Typically the methods of the present invention employ compositions including peroxyoctanoic acid, peroxynonanoic acid, or peroxyheptanoic acid, preferably peroxyoctanoic acid. Peroxyoctanoic (or peroctanoic) acid is a peroxycarboxylic acid having the formula, for example, of n-peroxyoctanoic acid: CH₃(CH₂)₆OOOH. Peroxyoctanoic acid can be an acid with a straight chain alkyl moiety, an acid with a branched alkyl moiety, or a mixture thereof. Peroxyoctanoic acid can be prepared through any number of methods known to those of skill in the art. A solution of peroxyoctanoic acid can be obtained by combining octanoic acid and hydrogen peroxide.

The compositions of the present invention preferably include only ingredients that can be employed in food products or in food transport, handling, or processing, for example, according to government (e.g. FDA or EPA) rules and regulations. In addition, the present compositions are preferably free of a coupling agent. Preferably, the composition is free of any peroxycarboxylic acid or carboxylic acid with 10 or more carbon atoms. Such 10 or more carbon acids can impart undesirable residues (e.g. bad tasting and/or malodorous) to a food product.

### Hydrogen Peroxide

The biocidal composition useful in methods of the invention may also include a hydrogen peroxide constituent. Hydrogen peroxide in combination with the percarboxylic acid provides a surprising level of biocidal action against microorganisms despite the presence of high loadings of organic sediment. Additionally, hydrogen peroxide can provide an effervescent action that can irrigate any surface to which it is applied. Hydrogen peroxide works with a mechanical flushing action once applied which further cleans the surface of application. An additional advantage of hydrogen peroxide is the food compatibility of this composition upon use and decomposition. For example, combinations of peroxyacetic acid and hydrogen peroxide result in acetic acid, water, and oxygen upon decomposition all of which are food product compatible.

While many oxidizing agents can be used, hydrogen peroxide is generally preferred for a number of reasons. After application of the H₂O₂/peroxyacetic acid germicidal agent, the residue left merely includes water and an acidic constituent. Deposition of these products on the surface of an apparatus, such as a knife, will not adversely affect the apparatus, the handling or processing, or the food products contacted.

Hydrogen peroxide, (H₂O₂) has a molecular weight of 34.014 and it is a weakly acidic, clear, colorless liquid. The four atoms are covalently bonded in an H--O--O--H structure. Generally, hydrogen peroxide has a melting point of -0.41degrees C, a boiling point of 150.2 degrees C, a density at 25 degrees C of 1.4425 grams per cm³, and a viscosity of 1.245 centipoise at 20 degrees C.

### Carrier

The biocide useful in the method of the invention may also include a carrier. The carrier provides a medium that dissolves, suspends, or carries the other components of the composition. For example, the carrier can provide a medium for solubilization and production of peroxycarboxylic acid and for forming an equilibrium mixture. The carrier also functions to deliver and wet the antimicrobial composition of the invention to the instrument. To this end, the carrier may contain any component or components that can facilitate these functions.

Generally, the carrier includes primarily water that is an excellent solubilizer and medium for reaction and equilibrium. The carrier can include or be primarily an organic solvent, such as simple alkyl alcohols, e.g., ethanol, isopropanol, n-propanol, and the like. Polyols are also useful carriers, including propylene glycol, polyethyleneglycol, glycerol, sorbitol, and the like. Any of these compounds may be used singly or in combination with other organic or inorganic constituents or, in combination with water or in mixtures thereof.

Generally, the carrier makes up a large portion of the biocide and may be the balance of the composition apart from the active antimicrobial components, adjuvants, and the like. Here again, the carrier concentration and type will depend upon the nature of the biocide as a whole, the environmental storage, and method of application including concentration of the antimicrobial agent, among other factors. Notably the carrier should be chosen and used at a concentration that does not inhibit the antimicrobial efficacy of the active agent in the biocide.

### Adjuvants

The biocidal composition employed in the method of the invention can also include any number of adjuvants. Specifically, the composition useful in the method of the invention can include stabilizing agents, wetting agents, as well as pigments or dyes among any number of constituents which can be added to the composition.

Stabilizing agents can be added to the biocide to stabilize the peracid and/or the hydrogen peroxide and prevent the premature oxidation of this constituent within the biocide. Chelating agents or sequestrants generally useful as stabilizing agents in the biocide include alkyl diamine polyacetic acid-type chelating agents such as EDTA (ethylene diamine tetra acetate tetrasodium salt), acrylic and polyacrylic acid-type stabilizing agents, phosphonic acid, and phosphonate-type chelating agents among others. Preferable sequestrants include phosphonic acids and phosphonate salts including 1-hydroxy ethyldene-1,1-diphosphonic acid, amino[tri(methylene phosphonic acid)], 2-phosphene butane-1,2,4-tricarboxylic acid, as well as the alkyl metal salts, ammonium salts, or alkyloyl amine salts, such as mono, di, or tetra-ethanolamine salts. The stabilizing agent is used in a concentration ranging from about 0 weight percent to about 20 weight percent of the composition, preferably from about 0.1 weight percent to about 10 weight percent of the composition, and most preferably from about 0.2 weight percent to 5 weight percent of the composition.

Also useful in the biocides useful in the method of the invention are wetting and defoaming agents. Wetting agents function to increase the penetration activity of the biocide. Wetting agents that can be used in the biocides useful in methods of the invention include any of those constituents known within the art to raise the surface activity of the biocide.

Along these lines surfactants, and especially nonionic surfactants, can also be useful in the biocide. Nonionic surfactants which can be useful in the present invention are those which include ethylene oxide moieties, propylene oxide moieties; as well as mixtures thereof, and ethylene oxide-propylene oxide moieties in either heteric or block formation. Additionally useful in the biocide are nonionic surfactants which include an alkyl ethylene oxide compounds, alkyl propylene oxide compounds, as well as mixtures thereof, and alkyl ethylene oxide-propylene oxide compounds where the ethylene oxide propylene oxide moiety is either in heteric or block formation. Further useful in the biocide are nonionic surfactants having any mixture or combination of ethylene oxide-propylene oxide moieties linked to an alkyl chain where the ethylene oxide and propylene oxide moieties can be in any randomized or ordered pattern and of any specific length. Nonionic surfactants useful in the biocide can also include randomized sections of block and heteric ethylene oxide propylene oxide, or ethylene oxide-propylene oxide.

Generally, the concentration of nonionic surfactant used in a biocide useful in the method of the invention can range from about 0 wt-% to about 5 wt-% of the composition, preferably from about 0 wt-% to about 2 wt-% of the concentrate composition, and most preferably from about 0 wt-% to about 1 wt-% of the composition.

The composition used in the methods of the invention can also contain additional ingredients as necessary to assist in defoaming. Generally, defoamers useful in accordance with the invention include silica and silicones; aliphatic acids or esters; alcohols; sulfates or sulfonates; amines or amides; halogenated compounds such as fluorochlorohydrocarbons; vegetable oils, waxes, mineral oils as well as their sulfated derivatives; fatty acid soaps such as alkali, alkaline earth metal soaps; and phosphates and phosphate esters such as alkyl and alkaline diphosphates, and tributyl phosphates among others; and mixtures thereof.

Especially useful are those antifoaming agents or defoamers that are of food grade quality given the application of the method of the invention. To this end, one of the more effective antifoaming agents includes silicones. Silicones such as dimethyl silicone, glycol polysiloxane, methylphenol polysiloxane, trialkyl or tetralkyl silanes, hydrophobic silica defoamers and mixtures thereof can all be used in defoaming applications. Commercial defoamers commonly available include silicones such as Ardefoam® from Armour Industrial Chemical Company which is a silicone bound in an organic emulsion; Foam Kill® or Kresseo® available from Krusable Chemical Company which are silicone and non-silicone type defoamers as well as silicone esters; and Anti-Foam A™ and DC-200 from Dow Corning Corporation which are both food grade type silicones among others. These defoamers can be present in the biocide at a concentration range from about 0.01 wt-% to 5 wt-%, preferably from about 0.01 wt-% to 2 wt-%, and most preferably from about 0.01 wt-% to about 1 wt-%.

The biocide useful in the method of the invention can also contain any number of other constituents as necessitated by the application, which are known to those of skill in the art and which can facilitate the activity of the present invention. In a preferred embodiment, biocides useful in the methods of the invention are free of a coupling agent.

The present methods require a certain minimal contact time of the composition with a microbe for occurrence of antimicrobial activity. Preferably the exposure time is at least about 1.5 seconds, at least about 2 seconds. The amount of reduction of microbial organisms can vary according to the conditions of use such as: concentration of peroxy acid in the use composition, temperature, and exposure time.

The method of the invention is effective at reducing bacterial loads and/or sterilizing slaughtering and butchering equipment including knives with as little contact time as 1 second at a temperature as low as 11 degrees Centigrade. In an embodiment, the contact time is increased to 1.5 seconds, 2.0 seconds. However, the increased contact time is unnecessary to produce sterile equipment and cutting surfaces. The skilled artisan will recognize the benefit in applying the biocide at the lowest temperature possible. A lower temperature requires less energy because heating of the biocide to a lower temperature is less expensive than heating the biocide to a higher temperature.

An advantage of practicing methods of the invention includes that post-use rinse of the cutting instrument or knife is not required. That is, once the cutting instrument is contacted with the biocide according to the prescribed times and temperatures, no further rinse of the instrument is necessary. Therefore, there is no need to employ an additional potable water flush of the cutting instrument. This together with lower throughput requirement to keep the sterilization water at a certain temperature, is a distinct advantage of the invention since water consumption is dramatically reduced. Moreover, energy consumption is dramatically reduced because there is no need to heat any water for a hot water rinse.

Methods of the invention include applying the biocide to a knife or cutting instrument used in processing food. In particular, methods of the invention are employed in animal processing plants. By way of further example, a biocide can be applied to knives and cutting instruments or tools before or during processing poultry to reduce the microbial load on the instruments and thereby reduce the microbial load on the carcass or meat in general. The mixture of organic acids containing a fatty acid is capable of removing fatty residues that normally build up during incisions without hot water rinse. In order to prevent build up of fatty residues on the surface of cutting tools, a temperature below 42°C is recommended. Any method is useful in applying the biocide to a knife or cutting instrument may be used in the present invention. Such application method includes but is not limited to immersing the cutting instrument in the biocide, spraying the biocide onto the surface, or pouring the biocide onto the surface of the cutting instrument. Spraying the biocide can include but is not limited to spraying through a nozzle or through an opening in the form of a mist or a stream. Any of these contacting or application methods may be used alone or in combination with another application means.

Without being bound by theory, it is believed that the method of the present invention may work by oxidizing the outer cellular membrane of micro-organisms. Alternatively, or in conjunction with the oxidization, a secondary theory of operability is the acidification of the blade(s) or cutting surface(s). A third theory of operability and efficacy and also a very important feature of the present method is its ability to prevent or inhibit formation of biofilms. That is, since no hot water is employed in the present invention, the protein coagulates are not formed on the cutting blades.

It is believed that by practicing the method of the present invention the bacterial counts, particularly those of Salmonella and coliform in addition to the total viable counts of microbes are overall reduced on carcasses processed using the method. Without being bound by theory it is believed that the result of this reduction in microbiological count or load is caused by decreasing cross contamination of cutting tools.

### Preparation of biocide

The biocide is diluted into a reservoir at a concentration of approximately 220 ml/L. As earlier stated, in order to comply with food processing standards, the concentration of peroxyacetic acid is preferably less than 220 ppm. Blending proceeds during static mixing of the biocide. All contact parts for mix tanks, raw material holding tanks, pumps, valves and piping are ideally constructed of plastic, non-metallic or peroxyacid approved materials. The dosing unit and centrifugal pumps are preferably equipped with automatic degassing valves that operate as oxygen gas inevitably builds up during production. In order to remove oxygen from the concentrate drums, the concentrate is preferably recycled in the dosing unit and depressurized during non-operational hours. The biocide may be applied at pressures up to 250 psi, up to about 200 psi, up to about 150 psi at temperatures up to about 45°C.

Storage of the prepared biocide is important. If long term storage is necessary, it is desirable to store the biocide in a cool and dark environment so as not to degrade the oxidizing agents of the biocide. It is believed that temperatures exceeding about 30 degrees C cause degradation of the biocide, temperatures above about 25 degrees C may cause degradation of the biocide. If long term storage, that is, exceeding a few days, is necessary, a cool dark environment may be preferable for maintaining the effectiveness of the biocide.

The present invention may be better understood with reference to the following examples. These examples are intended to be representative of specific embodiments of the invention, and are not intended as limiting the scope of the invention.

### Examples

Biocide A was prepared by combining 30 % w/w hydrogen peroxide, 35% (H₂O₂) with 59% w/w glacial acetic acid, 10% w/w octanoic or caprylic acid, 100% (CH₃(CH₂)₆COOH) and 1% w/w 1-hydroxyethelidene-1,1-diphosphonic acid, 60% (HEDP) at 70-80F. Measured quantities of each of the above components were added in the order provided to a high density polyethylene (HDPE) mix tank. Mixing continued for 60 minutes and equilibrium was allowed to set for approximately 1 week at ambient temperature. Biocide A was used in the following examples.

### Example 1

Diluted Biocide A (0.15%; 210 ppm), was stored in a buffer tank where it was ready for use before pumping with centrifugal pumps to slaughter robots used in slaughtering pigs. Normally, the slaughter robots are connected with hot water and cold water connections. In order to practice the method of the invention, hot water connections were replaced by biocide connections. This allowed spraying of the biocide onto the cutting instruments through nozzles. One centrifugal pump (3 bar outlet pressure) was used for every slaughter robot (1 bar hot water inlet pressure). In this Example, 2 centrifugal pumps were used. To obtain 1 bar inlet pressure, the hose connection between the centrifugal pump toward the slaughter robot should not exceed 20 m. For larger distances, fixed (metal) lines or heavier centrifugal pumps are required. No manual activation of the mixing and dispensing machine was necessary. Upon activation of the bung dropper and carcass splitter, the automatic mixing and dispensing of biocide was activated as well. When the machines were turned off, the biocide dispensing machine was turned off automatically as well due to increase of internal pressure (pressure switch in the centrifugal pump). A trial was conducted to compare the method of the present invention to the previously known method of disinfecting cutting tools. That is, the method of applying a biocide comprising an organic acid containing a fatty acid to a cutting instrument for 1 second at a temperature of about 15 degrees was compared to the standard procedure of disinfecting with 82°C water.

The trial was divided into three periods of two weeks where one week consisted of Monday through Friday. During the first trial period (referred to hereafter as "Reference Period 1"), the slaughter-robots were disinfected with the standard procedure of 82°C water. In Reference Period 2, which was actually the Test Period, Biocide A was used in accordance with the method of the invention. In the third and last period ("Reference Period 3") the standard method was applied again. The reference measurements were performed to compare the effectiveness of the current method against the present invention. The Third Reference Period was intended to observe if any effect of the Test Period, also referred to as Reference Period 2, was permanent.

The trial slaughterhouse had a slaughter capacity of 6000 pigs per day with one slaughter-line accommodating approximately 750 carcasses /hour. Working hours were from +/- 5 a.m. to 4 p.m. A lunch break was held at about 1:00 p.m. during which the borer (bit) of the bung dropper was sharpened as well as the blades of the carcass-splitter. Both machines were normally rinsed with water during the break. After production, the total evisceration area was thoroughly sanitized according to European hygiene regulations and the slaughterhouse quality assurance protocol.

During the trial, microbiological samples were taken from the cutting blades of the bung dropper and carcass splitter. Microbiological samples were taken from carcasses as described and respective monitoring- and validation logs were collected during the day. Monitoring began before production and ended after production.

Monitoring was performed after carcass splitting and during inspection of the carcasses. Evaluation of residues on the hide was undertaken to observe whether residues of Biocide A were present on the carcasses. Samples were taken with a cork borer. One sample of the rind side and one sample of the meat side of the carcass were taken. Two samples from each carcass were analysed in distilled water after stirring a Merckoquant-test strip. The procedure is outlined more fully below.

Cork-borer hide samples were taken from rind of the dorsal side of the carcass (10 cm below the ham; 5 cm from incision area). Cork borer hide samples were thoroughly flushed with 10 cm water in a plastic beaker. Concentration of peroxyacetic acid was measured by a Merckoquant test strip (range 5 mg/L - 50 mg/L). The Merckoquant test strips are most practical in a slaughter environment where a fast and efficient measurement without the availability of lab equipment is necessary. The strip was not directly used on carcasses. To measure the amount of peroxyacids on the carcass, 10 mls distilled water rinse solution directly applied to the carcass was measured. Any peroxyacids present dissolve; therefore, to find the actual amount of peroxyacids, the results were multiplied by 10. Consequently, a residue of 50 mg/L on the carcass was measured with a test strip. Since organic material has a diminishing effect on any residues, lowering the amount of rinse fluid increases organic load thereby resulting in invalid measurements.

The samples were taken the first three days of the experiment and the first day of the second week. Each day, potential residues on the carcass were monitored 4 times. If no residues were found, samples were reduced to 2 times per day during the remainder of the experiment.

Microbiological evaluation: The two bung droppers and two carcass splitters in the slaughterhouse that were observed during the trial were sampled three times per day. In the morning before slaughter (± 5 am), in the afternoon during the lunch break (± 1 pm) and at the end of the day after the last pigs were slaughtered (± 4 pm). Both the bung droppers and carcass splitters were sampled on five and ten positions respectively. The agar plates (3 cm) for testing of total aerobe count and *enterobacteriaceae* were pressed on sample places next to each other, to prevent overlap. Plate Count Agar (PCA) was used for the determination of the mesophilic aerobic counts and Violet Red Bile Glucose (VRBG) agar for the *enterobacteriaceae* counts.

The agar samples taken on the slaughter robots were taken from the supposed critical areas which come into direct contact with the carcass and/or may be regarded as a potential hazard in propagating contaminating micro-organisms. The locations tested on the bung droppers were: the drill chucks, style below, style above ball, bit centre, bit below. The locations tested on the carcass splitters are: diabolo roll (yellow), knife left front, knife left back, knife right front, knife right back, metal roll left, knife holder left, top of the nozzles left, top of the nozzles right, inside the hood.

At the end of the day, after the last slaughtered pig (± 4 pm), a sponge sample was taken to determine the absence or presence of *Salmonella* species. For this method a sponge was moisturized (Nasco, Speci-Sponge^{®} including 25 ml buffered peptone water) and pulled over the sample surface. Plastic gloves (one per sample) and a sealed spongebag (Nasco, Whirl-Pack^{®}) were used to prevent contamination of the sample. From the two bung droppers and two carcass splitters, two and three samples were taken, respectively. One sponge sample was regarded as a pooled sample for a larger surface area.

The carcasses in the chiller area were sampled every morning of the trial. The carcasses of the preceding slaughter day were sampled. On every first day of the week (Monday morning) carcass samples of the previous last day of the week (Friday afternoon) were taken. One carcass was randomly sampled from each group of 500 carcases. Each day a minimum of 12 carcasses were sampled. Hide microbiological samples for whole carcass analysis were taken with a cork borer from the following areas: ham area, the middle area, the front area above the shoulder and the jug. One carcass sample; therefore, consisted of four meat samples that were pooled.

After sampling for mesophilic counts, the PCA plates (sampled from slaughter-robots), were incubated for 72 ± 3 hour at 30 ± 1°C. The VRBG agar plates for *enterobacteriaceae* counts were incubated at 37 ± 1°C for 24 ± 1 hour. Enumeration of *enterobacteriaceae* and mesophilic aerobic colonies on the respective agar plates was performed. Agar counts per cm² were ¹⁰log transformed. The detection level for the agar samples was -0.89 ¹⁰log since 1 colony forming unit ("cfu") was the lowest number of *enterobacteriaceae* or mesophilic aerobic count that could be detected on agar plates. The analysis of the sponge samples on *Salmonella* species (sampled from slaughter-robots) was performed. The outcome of this analysis on *Salmonella* was binary: either present or absent. Quantification of Salmonella was not possible since enumerations were below detection level (<30 cfu/cm²).

Carcass samples were analyzed for mesophilic aerobic count, *enterobacteriaceae* and *Salmonella. Salmonella* determination was qualitative. Mesophilic aerobic and *enterobacteriaceae* counts from carcass samples were log transformed in the same manner as the slaughter-robot data was log transformed.

### Microbiological Results

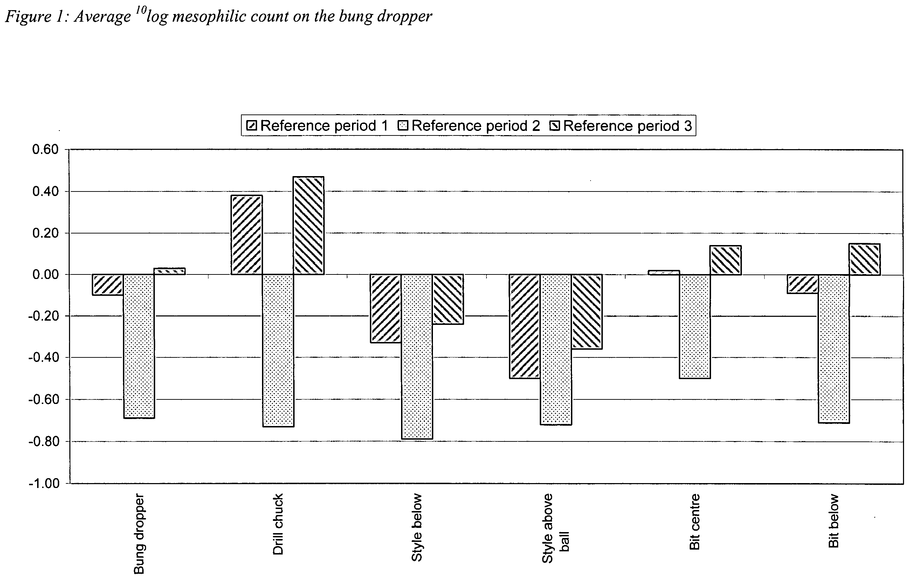

Figure 1 shows the microbiological results for mesophilic bacteria during the three described Reference Periods on the bung dropper, drill chuck, the style below ball, the style above ball, the bit center, and the bit below. The Bung dropper consists of a cylindrical knife which is called the bit. The bit below refers to the surface of this cylindrical knife on the cutting edge, and the bit center refers to the middle surface of the cylindrical knife. The style below ball refers to a rod inside the cylindrical knife through which a vacuum removes faecal matter and stabilizes the anal cavity during drilling while the style above ball refers to the rest of the rod up to the drilling chuck. The drilling chuck is the actual base of both style and bit. The bung dropper is composed of all mentioned components including auxillary parts and equipment. The results show that the method of Reference Period 2 or the method of the invention is more effective at disinfecting the tested cutting surfaces than hot water (82 degrees C) disinfection.

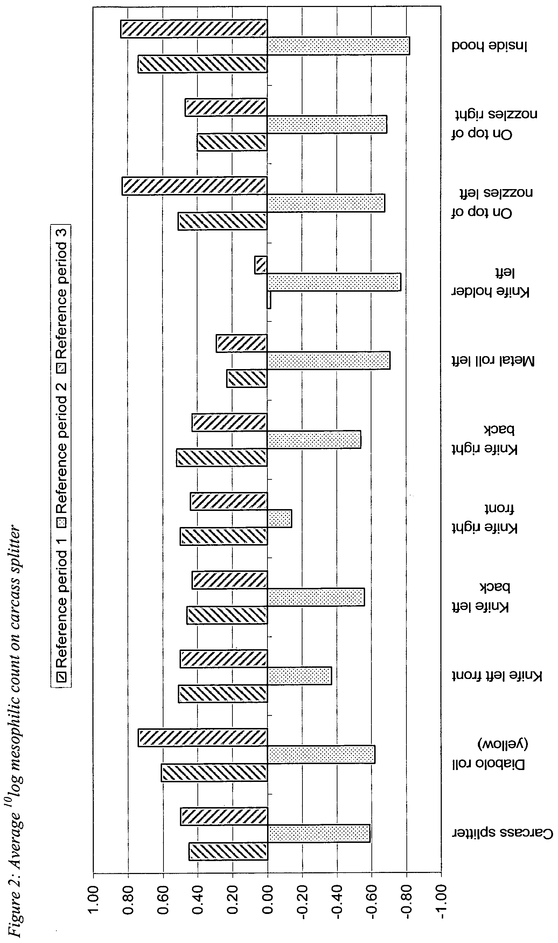

Figure 2 shows the microbiological results for mesophilic bacteria count during the three Reference Periods on various portions of the carcass splitter. The results show that the method of Reference Period 2 or the method of the invention is more effective at disinfecting the tested cutting surfaces than hot water (82 degrees C) disinfection. The carcass splitter (chopper type) consists of two large sliding blades (called left and right knifes) connected to the knife holder. The knife holder is usually mounted on a hydraulic installation. During operation, the knives chop alternatively in a downward fashion until the carcass is split vertically. One cycle consists of the knives pushed out of the manifold (hood) where in the prior art are disinfected by hot and cold water rinses (nozzles). A hydraulically operated arm extends forward and slides down to stabilize the carcass vertically. Two rollers (Diabolo and metal rollers) stabilize the carcass during the splitting action. When stabilized vertically, the alternatively sliding knives assume motion and split the carcass until a pre-programmed length. The hydraulic arm with Diabolo and metal roller retract and the knives are retracted into the manifold where they are rinsed automatically. The manifold containing the hydraulic arm and knives are transported upward to the starting position where the new cycle is initiated.

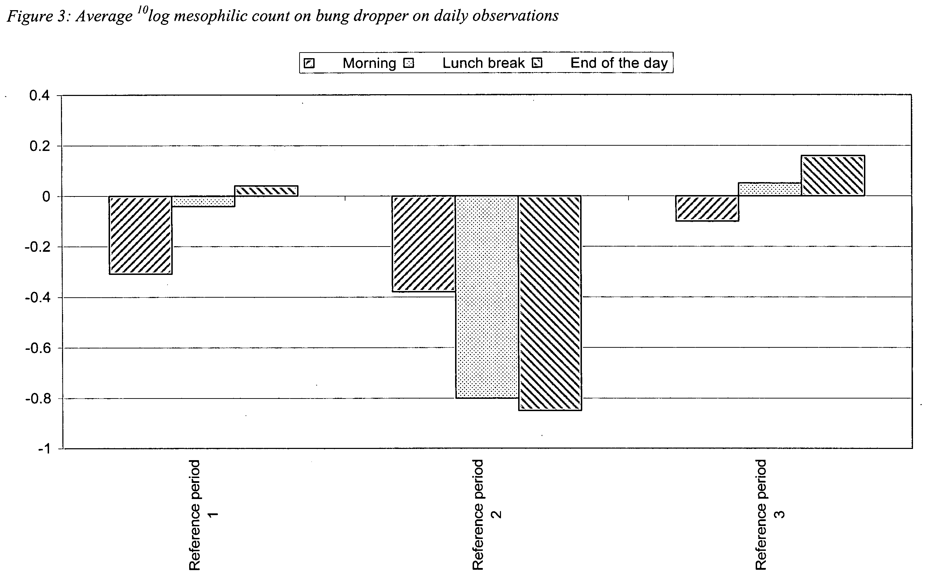

Figure 3 is a graphical representation of the count for mesophilic bacteria from the described referenced periods taken in the morning, at lunch break, and at the end of the day. The results show that the method of the invention (Reference Period 2 shown in the center of the graph) is a superior disinfecting method as compared to hot water disinfection.

Figure 3 is a graphical representation of the count for mesophilic bacteria from the described referenced periods taken in the morning, at lunch break, and at the end of the day. The results show that the method of the invention (Reference Period 2 shown in the center of the graph) is a superior disinfecting method as compared to hot water disinfection.

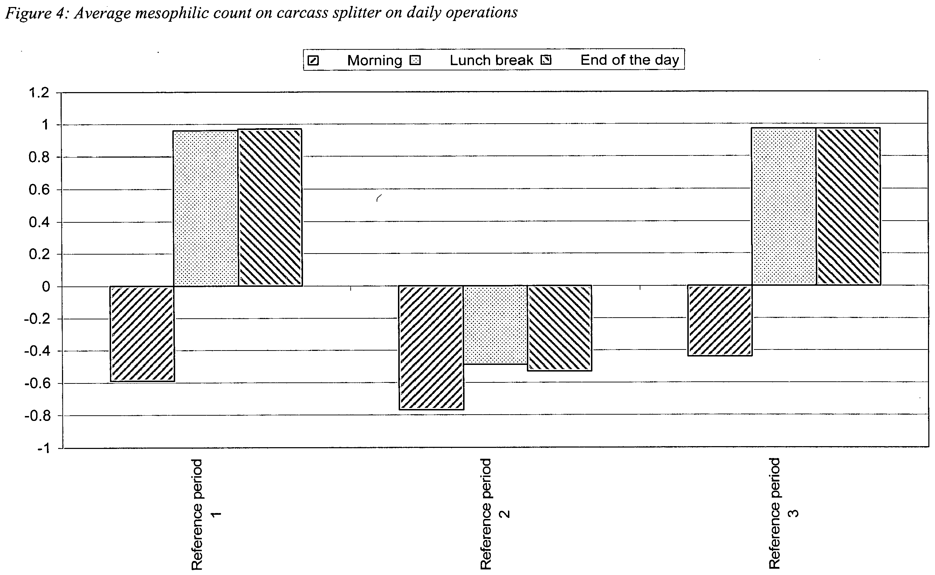

Figure 4 is a graphical representation of the count for mesophilic bacteria on the carcass splitter from the described referenced periods taken in the morning, at lunch break, and at the end of the day. The results show that the method of the invention (Reference Period 2 shown in the center of the graph) is a superior disinfecting method as compared to hot water disinfection.

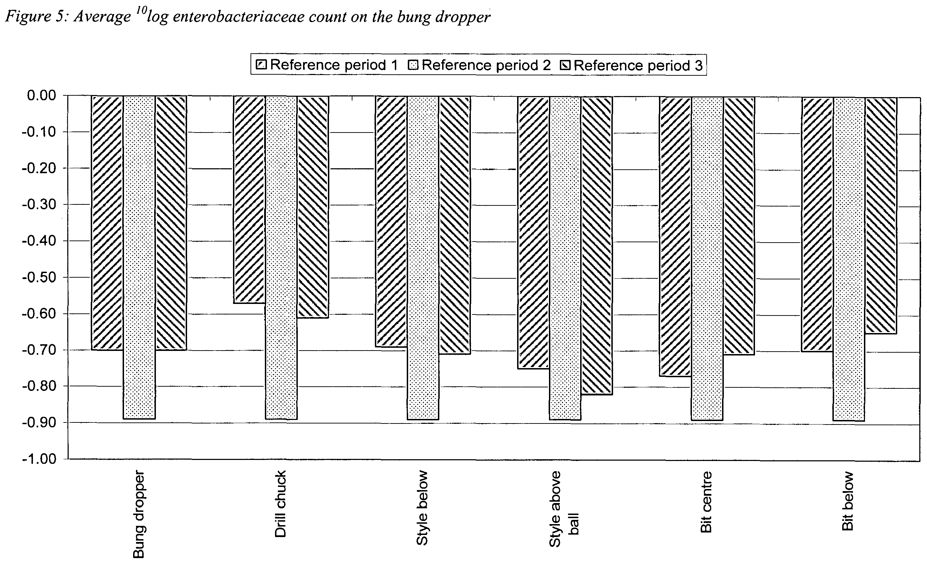

Figure 5 is a graphical representation of enterobacteriaceae count during the three described Reference Periods on the bung dropper, drill chuck, the style below ball, the style above ball, the bit center, and the bit below The results show that the method of Reference Period 2 or the method of the invention is more effective at disinfecting the tested cutting surfaces than hot water (82 degrees C).

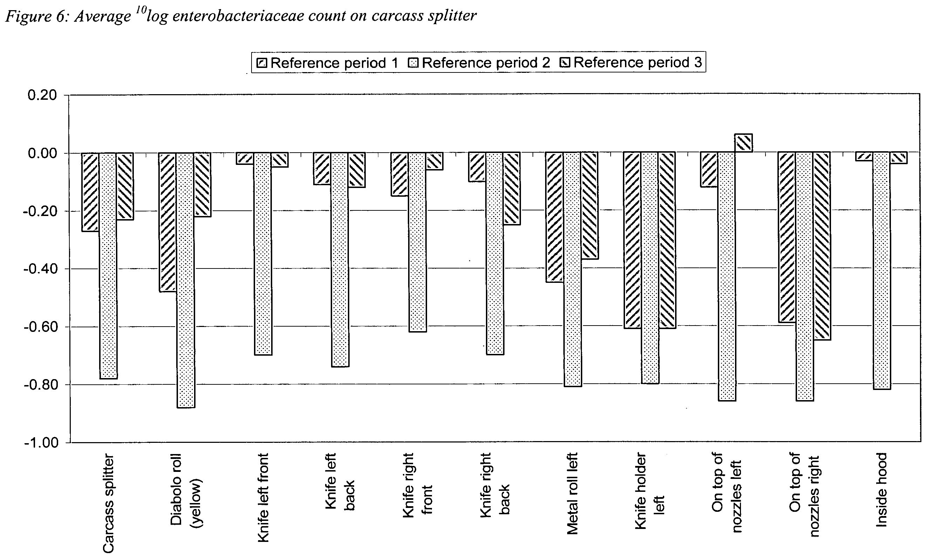

Figure 6 is a graphical representation of enterobacteriaceae count during the three described Reference Periods taken from various locations on the carcass splitter. The results show that the method of Reference Period 2 or the method of the invention is more effective at disinfecting cutting surfaces than hot water (82 degrees C).

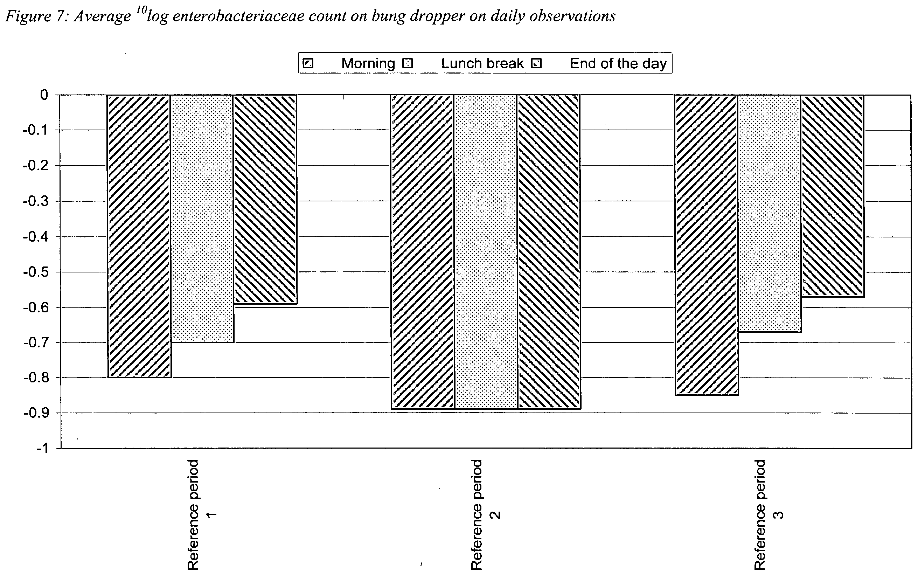

Figure 7 is a graphical representation of the count for enterobacteriaceae from the three described referenced periods taken in the morning, at lunch break, and at the end of the day. The results show that the method of the invention (Reference Period 2 shown in the center of the graph) is a superior disinfecting method as compared to hot water disinfection.

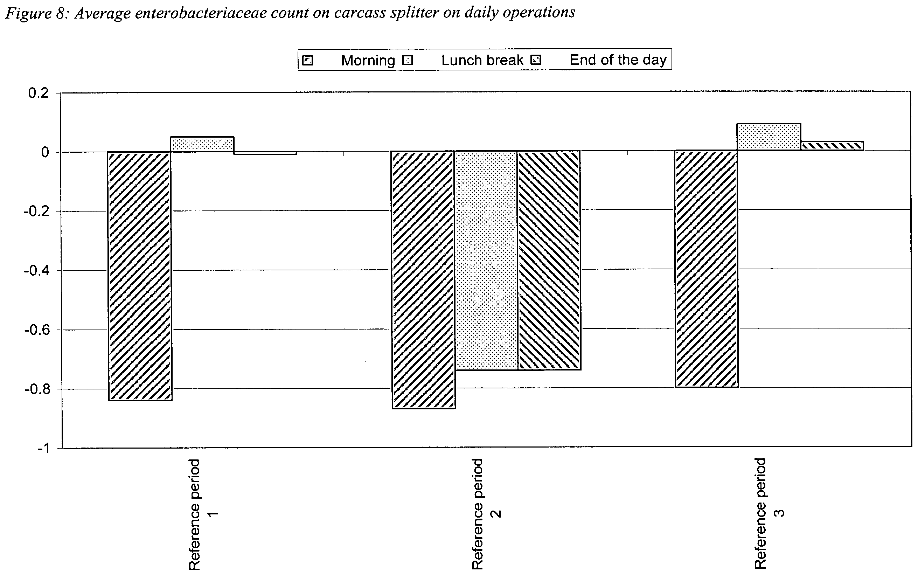

Figure 8 is a graphical representation of the count for enterobacteriaceae on the carcass splitter from the described referenced periods taken in the morning, at lunch break, and at the end of the day. The results show that the method of the invention (Reference Period 2 shown in the center of the graph) is a superior disinfecting method as compared to hot water disinfection.

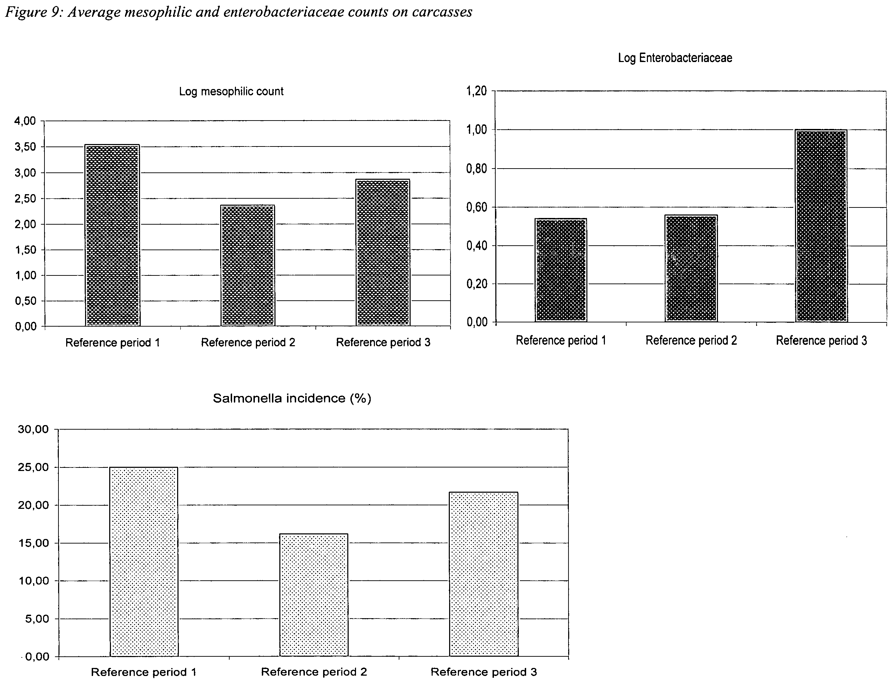

Figure 9 contains three graphs. The first graph is a representation of the average mesophilic bacteria count on carcasses taken during the three described Reference Periods. The second graph shows a representation of the average *enterobacteriaceae* count on carcasses taken during the three described Reference Periods. The third graph represents the percentage of *Salmonella* presence on carcasses taken during the three described Reference Periods. The results show that the method of Reference Period 2 or the method of the invention is more effective at reducing bacterial load on carcasses than hot water (82 degrees C) disinfection.

### Example 2

Residues of peroxyacid was measured on carcasses: In order to accomplish such measurement in a fast and efficient manner; a test-strip method was used. The test strip method indicated the remaining amount of peroxyacids as well as degradation of peroxaycids on the carcass. In order to validate the use of this method the actual degradation of peroxyacids on pig meat (5 g) was measured in a controlled laboratory trial. Pig rind was rinsed with 0.5, 1.0, 2.0, and 5.0 mls of Biocide A, having a composition as provided below, all containing 0.15% peroxyacid (220 ppm) respectively. The biocide was left on the pig rind for 1, 15, 30, and 60 minutes respectively. Subsequently, the pig rind was rinsed in 10 mls or 50 mls distilled water and measured with Mercoquant Peroxacid specific test strip (range 5 ppm - 50 ppm). The initial treatment refers to the initial concentration of peroxyacid in Biocide A.

**Results of the spiking experiment (Measured volume 10 mls; Concentration in ppm)**

| *Biocide A Treatment* | *Initial concentration* | *1 minute treatment* | *15 minute treatment* | *30 minute treatment* | *60 minute treatment* |
|---|---|---|---|---|---|
| 0.5 mls 0.15% | 11 | 5 | 0 | 0 | 0 |
| 1.0 mls 0.15% | 22 | 5 | 0 | 0 | 0 |
| 2.0 mls 0.15% | 44 | 15 | 5 | 0 | 0 |
| 5.0 mls 0.15% | 110 | 50 | 30 | 15 | 7.5 |

**Results of the spiking experiment (Measured volume 50 mls; Concentration in ppm)**

| *Biocide A Treatment* | *Initial concentration* | *1 minute treatment* | *15 minute treatment* | *30 minute treatment* | *60 minute treatment* |
|---|---|---|---|---|---|
| 0.5 mls 0.15% | 2.2 | 0 | 0 | 0 | 0 |
| 1.0 mls 0.15% | 4.4 | 0 | 0 | 0 | 0 |
| 2.0 mls 0.15% | 8.8 | 5 | 5 | 0 | 0 |
| 5.0 mls 0.15% | 22 | 20 | 20 | 15 | 10 |

The results show that even after contacting the pig rind directly with Biocide A, the peroxyacid in the biocide effectively degraded to levels suitable for human ingestion. Therefore, application of Biocide A to the cutting instrument will result in an even smaller lode of peroxyacid on the pork thereby allowing human consumption.

### Example 3

A 183 day stability storage test was performed at room temperature to study the stability of Biocide A. The stability data indicated relative stability of 70% active substances (peroxyacetic acid and peroxyoctanoic acid) at 183 days. For this Example it was important that the Biocide A concentrate was produced, stored, mixed and dispensed in peroxyacid compatible plastics in order to inhibit degradation. Such suitable plastics include but are not limited to PE or Teflon.

## Claims

1. A method of reducing microbes on cutting tools used in slaughtering animals and/or carcass processing comprising contacting the tools with a biocide comprised of at least 0.15 wt % organic acid containing a fatty acid at a temperature of 11 to 15 degrees C for 1.0 to 3.0 seconds.

2. The method of claim 1, wherein the organic acid containing a fatty acid is comprised of peroxycarboxylic acids.

3. The method of claim 1 wherein the organic acid containing a fatty acid comprises peroxyacetic acid and peroxyoctanoic acid.

4. The method of claim 1 wherein the biocide further comprises at least one additional organic acid and a scale inhibitor.

5. The method of claim 1 further comprising contacting the tools before and/or during carcass processing

6. The method of claim 1 wherein the biocide further comprises a carrier.

7. The method of claim 1 wherein the biocide is comprised of peroctanoic acid, peroxyacid, octanoic acid, acetic acid, hydrogen peroxide, and
hydroxyethylidene diphosphonic acid.

8. The method of claim 1 wherein the biocide is comprised of about 35 to about 45 weight-% acetic acid, about 5 to about 15 weight-% octanoic acid, about 3 to about 8 weight-% hydrogen peroxide, about 8 to about 16 weight-% peroxyacetic acid, about 1 to about 5 weight-% peroxyoctanoic acid, and about 0.1 to about 2 weight-% chelating agent; wherein the composition comprises at least about 1 part by weight of peroxyoctanoic acid for each about 5 parts of peroxyacetic acid.

9. The method of claim 1, wherein the biocide
comprises about 30 to about 60 weight-% acetic acid, about 1 to about 15 weight-% octanoic acid, about 2 to about 12 weight-% hydrogen peroxide, about 6 to about 16 weight-% peroxyacetic acid, and about 0.1 to about 5 weight-% peroxyoctanoic acid, and about 0.1 to about 2 weight-% chelating agent.

10. The method of claim 1, wherein the biocide composition comprises: at least about 2 ppm of one or more mono- or di-peroxycarboxylic acids having up to 6 carbon atoms; and at least 0.5 ppm of one or more carboxylic acids having up to 12 carbon atoms.

11. The method of claim 8, wherein the biocide composition comprises one or more peroxycarboxylic acids having from 2 to 6 carbon atoms and a peroxycarboxylic acid having from 7 to 12 carbon atoms.

12. The method of claim 1, wherein said "contacting" the cutting tool or instrument is comprised of washing, spraying, rinsing, pouring, or immersing or combination thereof.

13. The method of claim 1 wherein the biocide composition is comprised of about 133 ppm acetic acid, about 33 ppm octanoic acid, about 17 ppm hydrogen peroxide, about 40 ppm peroxyacetic acid, about 10 ppm peroxyoctanoic acid, and about 2 ppm chelating agent.

14. The method of claim 6 wherein the carrier is comprised of water.

## Patentansprüche

1. Verfahren zur Verminderung von Mikroben auf Schneidwerkzeugen, welche verwendet werden bei der Schlachtung von Tieren und/oder der Verarbeitung von Schlachtkörpern, umfassend das Inkontaktbringen der Werkzeuge mit einem Biozid, umfassend wenigstens 0,15 Gew.-% an organischer Säure, enthaltend eine Fettsäure, bei einer Temperatur von 11 bis 15 °C Celsius für 1,0 bis 3,0 Sekunden.

2. Verfahren nach Anspruch 1, wobei die eine Fettsäure enthaltende organische Säure Peroxycarbonsäuren umfasst.

3. Verfahren nach Anspruch 1, wobei die eine Fettsäure enthaltende organische Säure Peroxyessigsäure und Peroxyoctansäure umfasst.

4. Verfahren nach Anspruch 1, wobei das Biozid ferner wenigstens eine zusätzliche organische Säure und einen Kalkablagerungsinhibitor umfasst.

5. Verfahren nach Anspruch 1, ferner umfassend das Inkontaktbringen der Werkzeuge vor und/oder während der Verarbeitung von Schlachtkörpern.

6. Verfahren nach Anspruch 1, wobei das Biozid ferner einen Trägerstoff umfasst.

7. Verfahren nach Anspruch 1, wobei das Biozid Peroctansäure, Peroxysäure, Octansäure, Essigsäure, Wasserstoffperoxid und Hydroxyethylidendiphosphonsäure umfasst.

8. Verfahren nach Anspruch 1, wobei das Biozid etwa 35 bis etwa 45 Gew.-% Essigsäure umfasst, etwa 5 bis etwa 15 Gew.-% Octansäure, etwa 3 bis etwa 8 Gew.-% Wasserstoffperoxid, etwa 8 bis etwa 16 Gew.-% Peroxyessigsäure, etwa 1 bis etwa 5 Gew.-% Peroxyoctansäure sowie etwa 0,1 bis etwa 2 Gew.-% Chelatbildner; wobei die Zusammensetzung wenigstens etwa 1 Massenteil an Peroxyoctansäure für jeweils etwa 5 Teile Peroxyessigsäure umfasst.

9. Verfahren nach Anspruch 1, wobei das Biozid
etwa 30 bis etwa 60 Gew.-% Essigsäure umfasst, etwa 1 bis etwa 15 Gew.-% Octansäure, etwa 2 bis etwa 12 Gew.-% Wasserstoffperoxid, etwa 6 bis etwa 16 Gew.-% Peroxyessigsäure sowie etwa 0,1 bis etwa 5 Gew.-% Peroxyoctansäure und etwa 0,1 bis etwa 2 Gew.-% Chelatbildner.

10. Verfahren nach Anspruch 1, wobei die Biozid-Zusammensetzung umfasst: wenigstens etwa 2 ppm an einer oder mehreren Mono- oder Diperoxycarbonsäuren, aufweisend bis zu 6 Kohlenstoffatome; und wenigstens 0,5 ppm an einer oder mehreren Carbonsäuren, aufweisend bis zu 12 Kohlenstoffatome.

11. Verfahren nach Anspruch 8, wobei die Biozid-Zusammensetzung eine oder mehrere Peroxycarbonsäuren umfasst, aufweisend 2 bis 6 Kohlenstoffatome, sowie eine Peroxycarbonsäure, aufweisend 7 bis 12 Kohlenstoffatome.

12. Verfahren nach Anspruch 1, wobei das "Inkontaktbringen" des Schneidwerkzeugs oder -instruments Waschen, Besprühen, Abspülen, Übergießen oder Eintauchen, oder eine Kombination davon, umfasst.

13. Verfahren nach Anspruch 1, wobei die Biozid-Zusammensetzung etwa 133 ppm Essigsäure umfasst, etwa 33 ppm Octansäure, etwa 17 ppm Wasserstoffperoxid, etwa 40 ppm Peroxyessigsäure, etwa 10 ppm Peroxyoctansäure und etwa 2 ppm Chelatbildner.

14. Verfahren nach Anspruch 6, wobei der Trägerstoff Wasser umfasst.

## Revendications

1. Procédé de réduction de microbes sur des outils de découpe utilisés dans l'abattage des animaux et/ou la transformation des carcasses comprenant la mise en contact des outils avec un biocide comprenant au moins 0,15 % en poids d'un acide organique contenant un acide gras à une température de 11 à 15 degrés C pendant 1,0 à 3,0 secondes.

2. Procédé selon la revendication 1, dans lequel l'acide organique contenant un acide gras est constitué d'acides peroxycarboxyliques.

3. Procédé selon la revendication 1 dans lequel l'acide organique contenant un acide gras comprend de l'acide peroxyacétique et de l'acide peroxyoctanoïque.

4. Procédé selon la revendication 1 dans lequel le biocide comprend en outre au moins un acide organique additionnel et un anti-incrustant.

5. Procédé selon la revendication 1 comprenant en outre la mise en contact des outils avant et/ou pendant la transformation des carcasses.

6. Procédé selon la revendication 1 dans lequel le biocide comprend en outre un vecteur.

7. Procédé selon la revendication 1 dans lequel le biocide est constitué d'acide peroctanoïque, de peroxyacide, d'acide octanoïque, d'acide acétique, de peroxyde d'hydrogène et d'acide hydroxyéthylidène diphosphonique.

8. Procédé selon la revendication 1 dans lequel le biocide est constitué d'environ 35 à environ 45 % en poids d'acide acétique, d'environ 5 à environ 15 % en poids d'acide octanoïque, d'environ 3 à environ 8 % en poids de peroxyde d'hydrogène, d'environ 8 à environ 16 % en poids d'acide peroxyacétique, d'environ 1 à environ 5 % en poids d'acide peroxyoctanoïque et d'environ 0,1 à environ 2 % en poids d'un agent chélatant ; dans lequel la composition comprend au moins environ 1 partie en poids d'acide peroxyoctanoïque pour chaque environ 5 parties d'acide peroxyacétique.

9. Procédé selon la revendication 1, dans lequel le biocide comprend d'environ 30 à environ 60 % en poids d'acide acétique, d'environ 1 à environ 15 % en poids d'acide octanoïque, d'environ 2 à environ 12 % en poids de peroxyde d'hydrogène, d'environ 6 à environ 16 % en poids d'acide peroxyacétique et d'environ 0,1 à environ 5 % en poids d'acide peroxyoctanoïque et d'environ 0,1 à environ 2 % en poids d'un agent chélatant.

10. Procédé selon la revendication 1, dans lequel la composition de biocide comprend : au moins environ 2 ppm d'un ou plusieurs acides mono- ou diperoxycarboxyliques comportant jusqu'à 6 atomes de carbone ; et au moins 0,5 ppm d'un ou plusieurs acides carboxyliques comportant jusqu'à 12 atomes de carbone.

11. Procédé selon la revendication 8, dans lequel la composition de biocide comprend un ou plusieurs acides peroxycarboxyliques comportant de 2 à 6 atomes de carbone et un acide peroxycarboxylique comportant de 7 à 12 atomes de carbone.

12. Procédé selon la revendication 1, dans lequel ladite « mise en contact » de l'outil ou de l'instrument de découpe consiste en un lavage, une pulvérisation, un rinçage, un versage ou une immersion ou une combinaison de ceux-là.

13. Procédé selon la revendication 1 dans lequel la composition de biocide est constituée d'environ 133 ppm d'acide acétique, d'environ 33 ppm d'acide octanoïque, d'environ 17 ppm de peroxyde d'hydrogène, d'environ 40 ppm d'acide peroxyacétique, d'environ 10 ppm d'acide peroxyoctanoïque et d'environ 2 ppm d'un agent chélatant.

14. Procédé selon la revendication 6 dans lequel le vecteur est constitué d'eau.
